(19) Europäisches Patentamt
European Patent Office
Office européen des brevets

(11) **EP 3 571 984 B1**

(12) **EUROPEAN PATENT SPECIFICATION**

(45) Date of publication and mention of the grant of the patent:
**21.10.2020 Bulletin 2020/43**

(51) Int Cl.:
*A61B 5/021* (2006.01)        *A61B 5/022* (2006.01)
*A61B 5/02* (2006.01)         *A61B 5/00* (2006.01)
*G16H 10/60* (2018.01)        *G16H 50/50* (2018.01)
*G16H 50/70* (2018.01)

(21) Application number: **19179743.0**

(22) Date of filing: **28.01.2010**

(54) **SYSTEMS FOR CREATING A MODEL FOR USE IN DETECTING A PERIPHERAL ARTERIAL PRESSURE DECOUPLING IN A SUBJECT AND FOR DETECTING PERIPHERAL ARTERIAL PRESSURE DECOUPLING USING ARTERIAL PRESSURE WAVEFORM DATA AND SAID MODEL**

NACHWEIS VON GEFÄSSERKRANKUNGEN MIT ARTERIENDRUCK-WELLENFORMDATEN

DÉTECTION D'ÉTATS VASCULAIRES À L'AIDE DE DONNÉES DE FORME D'ONDE DE TENSION ARTÉRIELLE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **09.02.2009 US 151023 P**
**26.01.2010 US 694205**

(43) Date of publication of application:
**27.11.2019 Bulletin 2019/48**

(62) Document number(s) of the earlier application(s) in accordance with Art. 76 EPC:
**10738982.7 / 2 393 421**

(73) Proprietor: **Edwards Lifesciences Corporation Irvine, CA 92614 (US)**

(72) Inventors:
• **HATIB, Feras**
  **Irvine, CA California 92614 (US)**
• **ROTELIUK, Luchy D.**
  **Irvine, CA California 92614 (US)**

(74) Representative: **Eisenführ Speiser Patentanwälte Rechtsanwälte PartGmbB Postfach 31 02 60 80102 München (DE)**

(56) References cited:
**US-A1- 2002 111 554     US-A1- 2006 235 323
US-A1- 2008 015 451**

• **Benjamin Pratt ET AL: "Calculating Arterial Pressure-Based Cardiac Output Using a Novel Measurement and Analysis Method", internet Biomedical Instrumentation & Technology, vol. 41, no. 5 12 October 2007 (2007-10-12), pages 403-411, XP055086642, online DOI: http://dx.doi.org/10.2345/0899-8205(2007)4 1[403:CAPCOU]2.0.CO;2 Retrieved from the Internet: URL:http://www.researchgate.net/publicatio n/5853603_Calculating_arterial_pressure-ba sed_cardiac_output_using_a_novel_measurem e nt_and_analysis_method/file/79e415097ff64b 8867.pdf [retrieved on 2013-11-04]**
• **STEPHEN A WRIGHT ET AL: "Subclinical impairment of arterial mechanics in systemic lupus erythematosus identified by arterial waveform analysis", RHEUMATOLOGY INTERNATIONAL ; CLINICAL AND EXPERIMENTAL INVESTIGATIONS, SPRINGER, BERLIN, DE, vol. 27, no. 10, 14 March 2007 (2007-03-14), pages 961-968, XP019539885, ISSN: 1437-160X, DOI: 10.1007/S00296-007-0327-4**

**Description**

**BACKGROUND**

Indicators such as stroke volume (SV), cardiac output (CO), end-diastolic volume, ejection fraction, stroke volume variation (SVV), pulse pressure variation (PPV), and systolic pressure variations (SPV), among others, are important not only for diagnosis of disease, but also for "real-time," i.e., continual, monitoring of clinically significant changes in a subject. For example, health care providers are interested in changes in preload dependence, fluid responsiveness and volume responsiveness in both human and animal subjects. Few hospitals are therefore without some form of equipment to monitor one or more cardiac indicators in an effort to provide a warning that one or more of the indicated changes are occurring in a subject. Many techniques, including invasive techniques, non-invasive techniques, and combinations thereof, are in use and even more have been proposed in the literature.

**SUMMARY**

Multivariate statistical models for the detection of a vascular condition in a subject are described. The multivariate statistical models are based on arterial pressure waveform data from a first group of subjects that were experiencing the vascular condition and a second group of subjects that were not experiencing the vascular condition. The model provides a model output value that corresponds to a first established value for the arterial pressure waveforms of the first set of arterial pressure waveform data and a second established value for the arterial pressure waveform of the second set of arterial pressure waveform data.

Additionally, methods for creating such multivariate statistical models for use in detecting a vascular condition in a subject are described. These methods involve providing a first set of arterial pressure waveform data from a first group of subjects that were experiencing the vascular condition and providing a second set of arterial pressure waveform data from a second group of subjects that were not experiencing the vascular condition. Then building a multivariate statistical model based on the first and second sets of arterial pressure waveform data. The multivariate statistical model provides a model output value that corresponds to a first established value for the arterial pressure waveforms of the first set of arterial pressure waveform data and a second established value for the arterial pressure waveform of the second set of arterial pressure waveform data.

Further, methods for the detection of a vascular condition in a subject using these multivariate statistical models are described. These methods involve providing arterial pressure waveform data from the subject and applying a multivariate statistical model to the arterial pressure waveform data to determine a cardiovascular parameter. The multivariate statistical model is prepared from a first set of arterial pressure waveform data from a first group of subjects that were experiencing the vascular condition and a second set of arterial pressure waveform data from a second group of subjects that were not experiencing the vascular condition. The multivariate statistical model provides a cardiovascular parameter that corresponds to a first established value for the arterial pressure waveforms of the first set of arterial pressure waveform data and a second established value for the arterial pressure waveform of the second set of arterial pressure waveform data. Then the cardiovascular parameter is compared to a threshold value. If the cardiovascular parameter is equal to or greater than the threshold value, the subject is experiencing the vascular condition, and, if the cardiovascular parameter is less than the threshold value, the subject is not experiencing the vascular condition.

The journal publication from Benjamin Pratt ET AL: "Calculating Arterial Pressure-Based Cardiac Output Using a Novel Measurement and Analysis Method", Biomedical Instrumentation & Technology,vol. 41, no. 5 12 October 2007 (2007-10-12), pages 403-411 describes to a new arterial waveform analysis technique that relates the blood flow to the arterial pressure using a hemodynamic model.

**DESCRIPTION OF DRAWINGS**

Fig. 1 shows an example of a complex blood pressure curve over one beat-to-beat heart cycle.
Fig. 2 shows a discrete-time representation of the pressure waveform of Fig. 1.
Fig. 3 shows the area under the systolic portion of the arterial pressure waveform.
Fig. 4 shows the statistical distributions of the area under the systolic phase of the arterial pressure waveform for normal subjects and hyperdynamic subjects.
Fig. 5 shows the duration of the systole for an arterial pressure waveform.
Fig. 6 shows the statistical distribution of the duration of the systole of the arterial pressure waveform for normal subjects and hyperdynamic subjects.
Fig. 7 shows the duration of the systole and the duration of the diastole for an arterial pressure waveform.

Fig. 8 is the statistical distribution of the duration of the diastolic phase for high heart rate subjects in normal hemodynamic conditions (dashed line) and hyperdynamic conditions (thick line)-the distribution for all the patients combined is also shown (thin line).

Fig. 9 is the statistical distribution of the duration of the systolic phase for high heart rate subjects in normal hemodynamic conditions (dashed line) and hyperdynamic conditions (thick line)-the distribution for all the patients combined is also shown (thin line).

Fig. 10 shows simultaneously recorded pressure waveforms in the ascending aorta (Aortic), femoral artery (Femoral), and radial artery (Radial) in a porcine animal model during normal hemodynamic conditions.

Fig. 11 shows simultaneously recorded pressure waveforms in the ascending aorta (Aortic), femoral artery (Femoral), and radial artery (Radial) in a porcine animal model during Endotoxin shock (septic shock) resuscitated with large amounts of fluids and vasopressors.

Fig. 12 is a block diagram showing the main components of a system to implement the methods described herein.

## DETAILED DESCRIPTION

Multivariate statistical models for the detection of a vascular condition, methods for creating such multivariate statistical models, and methods for the detection of a vascular condition in a subject using the multivariate statistical models are described. The vascular condition may include different cardiovascular hemodynamic conditions and states, such as, for example, vasodilation, vasoconstriction, conditions where the peripheral pressure/flow is decoupled from central pressure/flow, conditions where the peripheral arterial pressure is not proportional to the central aortic pressure, and conditions where the peripheral arterial pressure is lower than the central aortic pressure.

The model for use in detecting a vascular condition described herein is a multivariate statistical model. This multivariate statistical model is based on arterial pressure waveform data from a first group of subjects that were experiencing a particular vascular condition and a second group of subjects that were not experiencing the same vascular condition. As an example, the first group of subjects that were experiencing a particular vascular condition are subjects that were experiencing decoupling of the peripheral arterial pressure/flow from the central aortic pressure/flow when their arterial pressure waveform data was collected. For further example, the second group of subjects that were not experiencing the same vascular condition were not experiencing decoupling of the peripheral arterial pressure/flow from the central aortic pressure/flow when their arterial pressure waveform data was collected, e.g., these subjects were experiencing normal vascular conditions. The multivariate statistical model is set up to provide different output values for each set of input data. Specifically, the multivariate statistical model provides a model output value that corresponds to a first established value for the arterial pressure waveforms of the first set of arterial pressure waveform data and a second established value for the arterial pressure waveform of the second set of arterial pressure waveform data.

The multivariate statistical model is based on a set of factors including one or more parameters affected by the vascular condition. Without wishing to be bound by theory, using one model and constraining the model to different output requirements for the first and second sets of data takes advantage of multiple factors to provide an indication that a vascular condition is occurring in a subject. Each type of factor used, e.g., pulse beats standard deviation, typically registers a difference between subjects experiencing a particular vascular condition and those not experiencing the condition. This difference, however, is often located along a continuum and a particular subject may have a value between a definite positive indication and a definite negative indication or for some reason in that subject the particular factor may appear to be within a normal range even though the subject is experiencing the vascular condition. However, by using multiple factors, i.e., multiple factors impacted by the vascular condition, there will typically be enough positive indications to indicate that a condition is present (or enough negative indications to indicate the condition is not present). Multivariate statistical models established as described herein provide the ability to use multiple factors to increase the ability to differentiate between two states, i.e., experiencing or not experiencing the vascular condition.

The specific number of factors used in a multivariate statistical model will depend on the ability of the individual factors to differentiate between a subject who is experiencing a particular condition and a subject who is not experiencing the particular condition. The number of factors can also be increased to provide a greater level of accuracy to a model. Thus, greater numbers of factors can be used to aid in the precision, accuracy, and/or reproducibility of a model as needed for particular circumstances. Examples of factors that can be used in the models described herein include (a) a parameter based on the pulse beats standard deviation of a set of arterial pressure waveform data, (b) a parameter based on the R-to-R interval (or the heart rate) of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data as defined by Langewouters et al. ("The Static Elastic Properties of 45 Human Thoracic and 20 Abnormal Aortas in vitro and the Parameters of a New Model," J. Biomechanics, 17(6):425-435 (1984)). Additional factors that can be used with the multivariate statistical models described herein include (n) a parameter based on the shape of the beat-to-beat arterial blood pressure signal and at least one statistical moment of the arterial blood pressure signal having an order of one or greater, (o) a parameter corresponding to the heart rate, and (p) a set of anthropometric parameters of the subject. While example of specific parameters are provided, in general, any time-domain, frequency-domain, or time-frequency domain parameters of the arterial pressure waveform can be used. One or more of these or other factors (or all of these or other factors) can be used in the multivariate statistical models described herein.

The factors used in the models and methods described herein are calculated from signals based on arterial blood pressure or signals proportional to, derived from, or a function of arterial blood pressure. The calculation of cardiovascular parameters, such as arterial compliance (arterial tone), is described in U.S. Patent Application Serial No. 10/890,887, filed July 14, 2004 Examples of factors and data used in calculating the cardiovascular parameters for use with the methods disclosed herein, including the parameters discussed in U.S. Patent Application Serial No. 10/890,887, are described below.

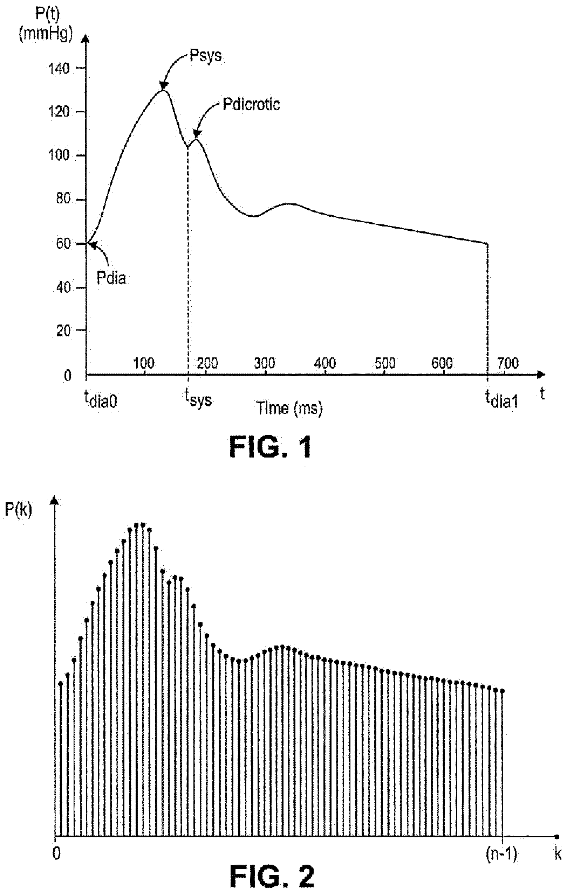

Fig. 1 is an example of an arterial pressure waveform, P(t), taken over a single heart cycle. This heart cycle starts at the point of diastolic pressure $P_{dia}$ at time $t_{dia0}$, through the time $t_{sys}$ of up to systolic pressure $P_{sys}$, to a time $t_{dia1}$ at which the blood pressure once again reaches $P_{dia}$.

Signals useful with the present methods include cardiovascular parameters based on arterial blood pressure or any signal that is proportional to, derived from, or a function of arterial blood pressure signal, measured at any point in the arterial tree, *e.g.,* radial, femoral, or brachial, either invasively or non-invasively. If invasive instruments are used, in particular, catheter-mounted pressure transducers, then any artery is a possible measurement point. Placement of non-invasive transducers will typically be dictated by the instruments themselves, e.g., finger cuffs, upper arm pressure cuffs, and earlobe clamps. Regardless of the specific instrument used, the data obtained will ultimately yield an electric signal corresponding (for example, proportional) to arterial blood pressure.

As illustrated in Fig. 2, analog signals such as arterial blood pressure can be digitized into a sequence of digital values using any standard analog-to-digital converter (ADC). In other words, arterial blood pressure, to ≤ t ≤ tf, can be converted, using known methods and circuitry, into the digital form P(k), k=0, (n-1), where $t_0$ and $t_f$ are initial and final times of the measurement interval and n is the number of samples of arterial blood pressure to be included in the calculations, distributed usually evenly over the measurement interval.

To capture relevant data from such digital or digitized signals, consider an ordered collection of m values, that is, a sequence Y(i), where i=1, ... , (m-1). As is well known from the field of statistics, the first four moments $\mu_1$, $\mu_2$, $\mu_3$, and $\mu_4$ of Y(i) can be calculated using known formulas, where $\mu_1$ is the mean (*i.e.,* arithmetic average), $\mu_2=\sigma^2$ is the variation (*i.e.,* the square of the standard deviation $\sigma$), $\mu_3$ is the skewness, and $\mu_4$ is the kurtosis. Thus:

$$\mu 1 = Yavg = 1/m * \Sigma(Y(i)) \qquad \text{(Formula 1)}$$

$$\mu 2 = \sigma^2 = 1/(m-1) * \Sigma(Y(i) - Y_{avg})^2 \qquad \text{(Formula 2)}$$

$$\mu 3 = 1/(m-1) * \Sigma[(Y(i) - Y_{avg})/\sigma]^3 \qquad \text{(Formula 3)}$$

$$\mu 4 = \sigma/(m-1) * \Sigma[(Y(i) - Y_{avg})/\sigma]^4 \qquad \text{(Formula 4)}$$

In general, the β-th moment $\mu_p$ can be expressed as:

$$\mu_\beta = 1(m-1) * 1/\sigma^\beta * \Sigma[(Y)(i) - Y_{avg})]^\beta \qquad \text{(Formula 5)}$$

where i=0, ... , (m-1). The discrete-value formulas for the second through fourth moments usually scale by 1/(m-1) instead of 1/m for well-known statistical reasons.

The methods described herein may utilize factors that are a function not only of the four moments of the pressure waveform P(k), but also of a pressure-weighted time vector. Standard deviation $\sigma$ provides one level of shape information in that the greater $\sigma$ is, the more "spread out" the function Y(i) is, *i.e.,* the more it tends to deviate from the mean. Although the standard deviation provides some shape information, its shortcoming can be easily understood by considering the following: the mean and standard deviation will not change if the order in which the values making up the sequence Y(i) is "reversed," that is, Y(i) is reflected about the i=0 axis and shifted so that the value Y($m$-1) becomes the first value in time.

Skewness is a measure of lack of symmetry and indicates whether the left or right side of the function Y(i), relative to the statistical mode, is heavier than the other. A positively skewed function rises rapidly, reaches its peak, then falls slowly. The opposite would be true for a negatively skewed function. The point is that the skewness value includes shape information not found in the mean or standard deviation values-in particular, it indicates how rapidly the function initially rises to its peak and then how slowly it decays. Two different functions may have the same mean and standard deviation, but they will then only rarely have the same skewness.

Kurtosis is a measure of whether the function Y(i) is more peaked or flatter than a normal distribution. Thus, a high kurtosis value will indicate a distinct peak near the mean, with a drop thereafter, followed by a heavy "tail." A low kurtosis value will tend to indicate that the function is relatively flat in the region of its peak. A normal distribution has a kurtosis of 3.0; actual kurtosis values are therefore often adjusted by 3.0 so that the values are instead relative to the origin.

An advantage of using the four statistical moments of the beat-to-beat arterial pressure waveform is that the moments are accurate and sensitive mathematical measures of the shape of the beat-to-beat arterial pressure waveform. As arterial compliance and peripheral resistance directly affect the shape of the arterial pressure waveform, the effect of arterial compliance and peripheral resistance could be directly assessed by measuring the shape of the beat-to-beat arterial pressure waveform. The shape sensitive statistical moments of the beat-to-beat arterial pressure waveform along with other arterial pressure parameters described herein could be effectively used to measure the combined effect of vascular compliance and peripheral resistance, *i.e.,* the arterial tone. The arterial tone represents the combined effect of arterial compliance and peripheral resistance and corresponds to the impedance of the well known 2-element electrical analog equivalent model of the Windkessel hemodynamic model, consisting of a capacitive and a resistive component. By measuring arterial tone, several other parameters that are based on arterial tone, such as arterial elasticity, stroke volume, and cardiac output, also could be directly measured. Any of those parameters could be used as factors in the models and methods described herein.

When the first four moments $\mu_{1P}$, $\mu_{2P}$, $\mu_{3P}$, and $\mu_{4P}$ of the pressure waveform P(k) are calculated and used in a model as described herein, where $\mu_{1P}$ is the mean, $\mu_{2P}$ P=$\sigma_P^2$ is the variation, that is, the square of the standard deviation $\sigma_P$; $\mu_{3P}$ is the skewness, and $\mu_{4P}$ is the kurtosis, where all of these moments are based on the pressure waveform P(k). Formulas 1-4 above may be used to calculate these values after substituting P for Y, k for i, and n for m.

Formula 2 above provides the "textbook" method for computing a standard deviation. Other, more approximate methods may also be used. For example, at least in the context of blood pressure-based measurements, a rough approximation to $\sigma_P$ is to divide by three the difference between the maximum and minimum measured pressure values, and that the maximum or absolute value of the minimum of the first derivative of the P(t) with respect to time is generally proportional to $\sigma_P$.

As Fig. 2 illustrates, at each discrete time k, the corresponding measured pressure will be P(k). The values k and P(k) can be formed into a sequence T(j) that corresponds to a histogram, meaning that each P(k) value is used as a "count" of the corresponding k value. By way of a greatly simplified example, assume that the entire pressure waveform consists of only four measured values P(1)=25, P(2)=50, P(3)=55, and P(4)=35. This could then be represented as a sequence T(j) with 25 ones, 50 twos, 55 threes, and 35 fours:

T(j)=1, 1, ... ,1,2,2, ..., 2, 3, 3, ..., 3, 4, 4, ...,4

This sequence would thus have 25+50+55+35=165 terms.

Moments may be computed for this sequence just as for any other. For example, the mean (first moment) is:

$$\mu_{1T}=(1*25+2*50+3*55+4*35)/165=430/165=2.606 \qquad \text{(Formula 6)}$$

and the standard deviation $\sigma_T$ is the square root of the variation $\mu_{2T}$:

$$\text{SQRT}[1/164*25(1-2.61)^2+50(2-2.61)^2+55(3-2.61)^2+35(4-2.61)^2]=0.985$$

The skewness $\mu_{3T}$ and kurtosis $\mu_{4T}$ can be computed by similar substitutions in Formulas 3 and 4:

$$\mu_{3T}=\{1/(164)*(1/\sigma_T{}^3)\Sigma[P(k)*(k-\mu_{1T})^3]\} \qquad \text{(Formula 7)}$$

$$\mu_{4T}=\{1/(164)*(1/\sigma_T{}^4)\Sigma[P(k)*(k-\mu_{1T})^4]\} \qquad \text{(Formula 8)}$$

where $k=1, \ldots , (m-1)$.

As these formulas indicate, this process in effect "weights" each discrete time value k by its corresponding pressure value P(k) before calculating the moments of time. The sequence T(j) has the very useful property that it robustly characterizes the timing distribution of the pressure waveform. Reversing the order of the pressure values P(k) will in almost all cases cause even the mean of T(j) to change, as well as all of the higher-order moments. Moreover, the secondary "hump" that normally occurs at the dicrotic pressure $P_{dicrotic}$ also noticeably affects the value of kurtosis $\mu_{4T}$; in contrast, simply identifying the dicrotic notch in the prior art, such as in the Romano method, requires noisy calculation of at least one derivative.

The pressure weighted moments provide another level of shape information for the beat-to-beat arterial pressure signal, as they are very accurate measures of both the amplitude and the time information of the beat-to-beat arterial pressure signal. Use of the pressure weighted moments in addition to the pressure waveform moments can increase the accuracy of the models described herein.

Additional parameters can be included in the computation to take other known characteristics into account, e.g., patient-specific complex pattern of vascular branching. Examples of additional values include body surface area BSA, or other anthropometric parameters of the subject, a compliance value C(P) calculated using a known method such as described by Langewouters et al. ("The Static Elastic Properties of 45 Human Thoracic and 20 Abnormal Aortas in vitro and the Parameters of a New Model," J. Biomechanics, 17(6):425-435 (1984)), which computes compliance as a polynomial function of the pressure waveform and the patient's age and sex, a parameter based on the shape of the arterial blood pressure signal and at least one statistical moment of the arterial blood pressure signal having an order of one or greater, a parameter based on the area under the systolic portion of the arterial blood pressure signal, a parameter based on the duration of the systole, and a parameter based on the ratio of the duration of the systole to the duration of the diastole.

These last three cardiovascular parameters, i.e., the area under the systolic portion of the arterial blood pressure signal, the duration of the systole, and the ratio of the duration of the systole to the duration of the diastole, are impacted by arterial tone and vascular compliance and, thus, vary, for example, between subjects in normal hemodynamic conditions and subjects in hyperdynamic conditions experiencing peripheral arterial pressure decoupling. Because these three cardiovascular parameters vary between normal and hyperdynamic subjects the methods described herein can use these cardiovascular parameters to detect vasodilation or vasoconstriction in the peripheral arteries of a subject.

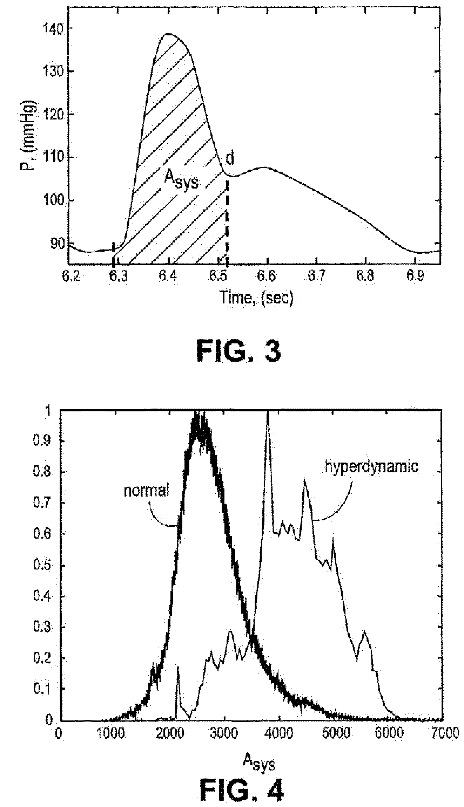

The area under the systolic portion of an arterial pressure waveform ($A_{sys}$) is shown graphically in Fig. 3. The area under the systolic portion of the arterial pressure waveform in an arterial pressure signal is defined as the area under the portion of the waveform starting from the beginning of the beat and ending in the dichrotic notch (from point b to point d on Fig. 3). The area under the systole represents the energy of the arterial pressure signal during systole, which is directly proportional to stroke volume and inversely proportional to arterial compliance. When measured over groups of normal and hyperdynamic patients a shift in $A_{sys}$ can be detected. As shown in Fig. 4, the energy of the arterial pressure signal during systole is higher, for example, in some subjects in hyperdynamic conditions. Those subjects with higher $A_{sys}$ are typically subjects with high cardiac output (CO) and low or normal HR, where the elevated CO is mainly caused by elevated heart contractility, which means that those subjects have increased stroke volume and decreased arterial compliance, which is directly reflected in the energy of the arterial pressure signal during systole. The reflected waves, which are usually very intense during many hyperdynamic conditions, may have also significant contribution to the increased energy of the signal during systole.

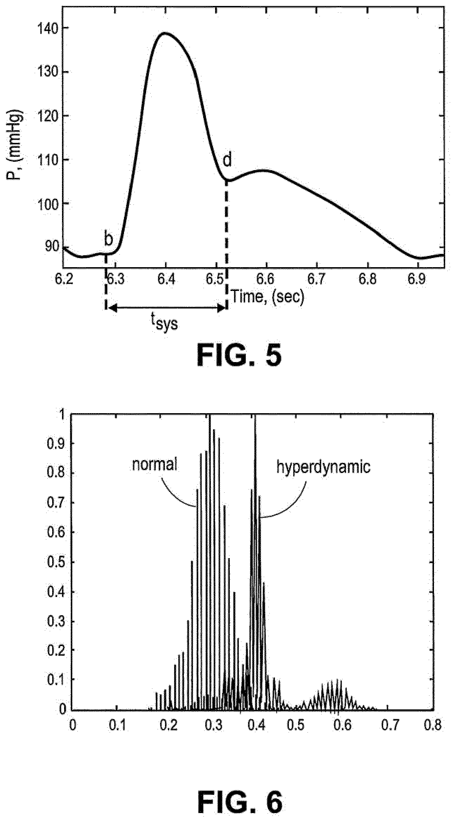

The duration of the systole ($t_{sys}$) is shown graphically in Fig. 5. The duration of the systole in an arterial pressure waveform is defined as the time duration from the beginning of the beat to the dichrotic notch (from point b to point d on Fig. 5). The duration of the systole is directly affected by the arterial compliance and is relatively independent of the changes in peripheral arterial tone, except when large reflect waves are present. As shown on Fig. 6, for example, the duration of the systole in some hyperdynamic subjects is higher than the duration of the systole in normal subjects (data shifted toward higher $t_{sys}$ values). As seen for the systolic energy, the duration of the systole is typically higher in patients with high CO who also have low or normal HR, where the elevated CO is mainly caused by elevated heart contractility and where the contractility may not have been high enough to increase the systolic energy. The increased stroke volume in those patients is partially due to increased contractility and partially due to increased duration of the systole. Reflected waves play a role here as well.

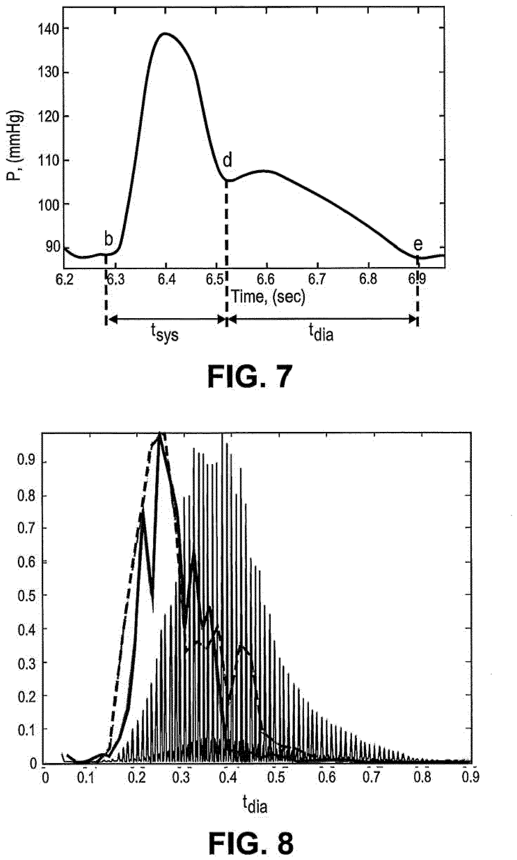

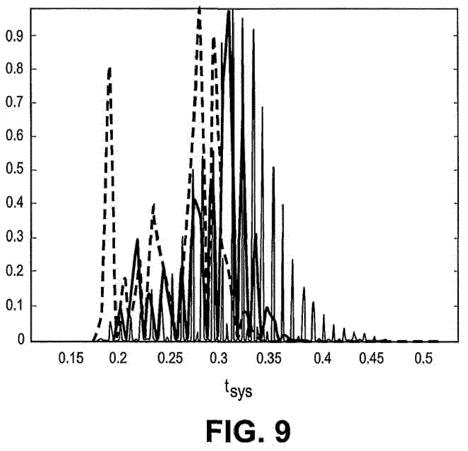

A further parameter that varies, for example, between normal and hyperdynamic subjects is the ratio of the duration of the systole ($t_{sys}$) and the duration of the diastole ($t_{dia}$), as shown graphically in Fig. 7. The duration of the diastole in an arterial pressure waveform is defined as the time duration from the dichrotic notch to the end of the cardiac cycle (from point d to point e on Fig. 7). In some hyperdynamic conditions, the ratio of the durations of the systole and diastole is significantly higher than that observed in normal hemodynamic conditions. This is typically observed in septic shock patients with elevated CO where HR is also high. In these types of conditions, the systole takes over almost the entire cardiac cycle leaving very little time for the diastole before the next cardiac cycle begins. This is shown in Figs. 8 and 9, which show the duration of diastole (Fig. 8) and the duration of systole (Fig. 9) during high HR conditions in septic shock patients and in normal patients. As shown in the figures, high HR patients in normal hemodynamic conditions (dashed line) tend to have low durations of both the systole and the diastole, while high HR patients in septic shock (thick line) tend to have low duration of the diastole but normal or high duration of the systole.

Other parameters based on the arterial tone factor such as, for example, Stroke Volume (SV), Cardiac Output (CO), Arterial Flow, Arterial Elasticity, or Vascular Tone can be used as factors in the models described herein.

The analog measurement interval, that is, the time window [to, tf], and thus the discrete sampling interval k=0, ... , (n-1), over which each calculation period is conducted should be small enough so that it does not encompass substantial shifts in the pressure and/or time moments. However, a time window extending longer than one cardiac cycle will provide suitable data. Preferably, the measurement interval is a plurality of cardiac cycles that begin and end at the same point in different cardiac cycles. Using a plurality of cardiac cycles ensures that the mean pressure value used in the calculations of the various higher-order moments will use a mean pressure value $P_{avg}$ that is not biased because of incomplete measurement of a cycle.

Larger sampling windows have the advantage that the effect of perturbations such as those caused by reflections are typically reduced. An appropriate time window can be determined using normal experimental and clinical methods well known to those of skill in the art. Note that it is possible for the time window to coincide with a single heart cycle, in which case mean pressure shifts will not be of concern.

The time window [$t_0$, $t_f$] is also adjustable according to drift in $P_{avg}$. For example, if $P_{avg}$ over a given time window differs absolutely or proportionally by more than a threshold amount from the $P_{avg}$ of the previous time window, then the time window can be reduced; in this case stability of $P_{avg}$ is then used to indicate that the time window can be expanded. The time window can also be expanded and contracted based on noise sources, or on a measure of signal-to-noise ratio or variation. Limits are preferably placed on how much the time window is allowed to expand or contract and if such expansion or contraction is allowed at all, then an indication of the time interval is preferably displayed to the user.

The time window does not need to start at any particular point in the cardiac cycle. Thus, to need not be the same as $t_{dia0}$, although this may be a convenient choice in many implementations. Thus, the beginning and end of each measurement interval (*i.e.,* to and tf) may be triggered on almost any characteristic of the cardiac cycle, such as at times $t_{dia0}$ or $t_{sys}$, or on non-pressure characteristics such as R waves, etc.

Rather than measure blood pressure directly, any other input signal may be used that is proportional to, derived from, or a function of blood pressure. This means that calibration may be done at any or all of several points in the calculations. For example, if a signal other than arterial blood pressure itself is used as input, then it may be calibrated to blood pressure before its values are used to calculate the various component moments, or afterwards, in which case either the resulting moment values can be scaled. In short, the fact that the cardiovascular parameter may in some cases use a different input signal than a direct measurement of arterial blood pressure does not preclude its ability to generate an accurate compliance estimate.

In one example, the decoupled output value for a multivariate Boolean model for a given set of factors is constrained to a first established value for those factors as obtained from the arterial pressure waveforms of the first set of arterial pressure waveform data and at the same time constrained to a second established value for those factors as obtained from the arterial pressure waveform of the second set of arterial pressure waveform data. The first established value and second established value can be set to provide a convenient comparison to model output values for a subject being analyzed. For example, the first established value can be greater than the second established value. Additionally, the first established value can be a positive number and the second established value can be a negative number. For further example, the first established value can be +100 and the second established value can be -100.

Such established values, i.e., +100 and -100, can be used to create a Boolean-type outcome for the multivariate statistical model. For example, for a multivariate statistical model with a first established value of +100 and a second established value of -100, the model output could be set up with the following indicators:

Model Output $\geq 0 \rightarrow$ vascular condition indicated

Model Output $< 0 \rightarrow$ vascular condition not indicated

In this case the "0" value can be considered a threshold value at or above which the vascular condition is indicated, i.e.,

values greater than or equal to zero more closely relate to the subjects used to establish the multivariate model that were experiencing the vascular condition than to those subjects that were not experiencing the vascular condition. Conversely, values lower than "0" indicate that the subject is experiencing vascular conditions more closely related to those subjects used to establish the multivariate model that were not experiencing the vascular condition than to those subjects that were experiencing the vascular condition. For this example, the threshold value was set at the mean between the first established value and the second established value. The threshold value could, however, be shifted based upon empirical observations. For example, if a value $\alpha$ is the mean value between the first and second threshold values, then the threshold value could be $\alpha$-1, $\alpha$-2, $\alpha$-3, $\alpha$-4, $\alpha$-5, $\alpha$-10, $\alpha$-15, $\alpha$-20, $\alpha$+1, $\alpha$+2, $\alpha$+3, $\alpha$+4, $\alpha$+5, $\alpha$+10, $\alpha$+15, or $\alpha$+20.

With some vascular conditions, there may be a range of threshold values that are not determinative of whether a subject is experiencing the vascular condition in question. In these cases, a threshold range can be used. For example, for a multivariate statistical model with a first established value of +100 and a second established value of -100, the model output could be set up with the following indicators:

Model Output $\geq$ 10 $\rightarrow$ vascular condition indicated

-10 < Model Output < 10 $\rightarrow$ indeterminate (continue to analyze)

Model Output $\leq$ -10 $\rightarrow$ vascular condition not indicated

In this example, values between 10 and -10 are considered indeterminate and further data can be analyzed to see if a value greater than or equal to 10 or less than or equal to -10 is indicated. Otherwise, for this situation, model output values greater than or equal to 10 indicate the vascular condition and values less than or equal to -10 indicate a normal vascular condition. The threshold range will depend upon an evaluation of the ability of the model to indicate a vascular condition at the intermediate point between the first and second established values. Additionally, the threshold range can be shifted or otherwise adjusted based upon empirical observations. For example, if a value $\alpha$ is the mean value between the first and second threshold values, then the threshold value could be $[(\alpha-1)\pm\beta]$, $[(\alpha-2)\pm\beta]$, $[(\alpha-3)\pm\beta]$, $[(\alpha-4)\pm\beta]$, $[(\alpha-5)\pm\beta]$, $[(\alpha-10)\pm\beta]$, $[(\alpha-15)\pm\beta]$, $[(\alpha-20)\pm\beta]$, $[(\alpha+1)\pm\beta]$, $[(\alpha+2)\pm\beta]$, $[(\alpha+3)\pm\beta]$, $[(\alpha+4)\pm\beta]$, $[(\alpha+5)\pm\beta]$, $[(\alpha+10)\pm\beta]$, $[(\alpha+15)\pm\beta]$, or $[(\alpha+20)\pm\beta]$, where $\beta$ is the upper and lower bounds of the range, e.g., $\beta$ can be 1, 2, 3, 4, 5, 6, 7, 8, 9, 10, 15, or 20 depending on the model.

Creating a multivariable statistical model to provide such Boolean results involves several steps. For example, a multiple linear regression response surface methodology can be used to establish the model. The number of terms used in the model can be determined using several numerical approaches to minimize the mean square error between the model output value and the values the model is forced to for the specific conditions. As a more detailed example, a predictor variable set for the model output value is related to a specific patient group, i.e., the established value can be set to +100 for subjects experiencing a specific vascular condition and can be set to -100 for subjects not experiencing the vascular condition. This operation creates a suite of reference values, each of which is a function of the component parameters of the model output value. A multivariate approximating function can then be computed, using known numerical models, that best relates the model outputs to either +100 or -100 depending on the subject group in the defined manner. A polynomial multivariate fitting function can then be used to generate the coefficients of the polynomial that satisfies the +100 and -100 constraints for each set of predictor values. Such a multivariate model (where $\chi$ is model output) has the following general form:

$$\chi = \begin{bmatrix} a_1 & a_2 & ... & a_n \end{bmatrix} * \begin{bmatrix} x_1 \\ x_2 \\ ... \\ x_n \end{bmatrix} \quad \text{(Formula 10)}$$

Where $a_1 ... a_n$ are the coefficients of the polynomial multi-regression model, and $x_1 ... x_n$ are the model's predictor variables. The predictor variables are selected from the factors discussed above that are derived from the arterial pressure waveforms.

Each of the model's predictor variables $x_i$ is a predefined combination of the arterial pressure waveform parameters $v_i$ and can be computed as follows:

$$x_i = \prod_m \left( \begin{bmatrix} v_1 & v_2 & \cdots & v_m \end{bmatrix}^{\wedge \begin{bmatrix} P_{1,1} & \cdots & P_{1,m} \\ \cdots & \cdots & \cdots \\ P_{n,1} & \cdots & P_{n,m} \end{bmatrix}} \right) \qquad \text{(Formula 11)}$$

The coefficients ai and the exponent matrix "P" can be determined by multivariate least-squares regression using the factor data collected from the subjects. They can be related, for example, to the +100 and -100 values depending on the patient's group for the population of reference subjects. For example, factor data from subjects experiencing a vascular condition can be related as follows:

$$\chi = \begin{bmatrix} a_1 & a_2 & \cdots & a_n \end{bmatrix} * \begin{bmatrix} x_1 \\ x_2 \\ \cdots \\ x_n \end{bmatrix} = +100 \qquad \text{(Formula 12)}$$

And factor data from subjects not experiencing a vascular condition can be related as follows:

$$\chi = \begin{bmatrix} a_1 & a_2 & \cdots & a_n \end{bmatrix} * \begin{bmatrix} x_1 \\ x_2 \\ \cdots \\ x_n \end{bmatrix} = -100 \qquad \text{(Formula 13)}$$

As a specific example, a multivariate model was created using 14 arterial pressure waveform factors (vi) in which 17 terms were required to provide a best fit for the model. These parameters were: $v_1$ (pulse beats standard deviation (std)), $v_2$ (R-to-R interval (r2r)), $v_3$ (area under the systole (sys area)), $v_4$ (the duration of the systole (t_sys)), vs (the duration of the diasystole (t_dia)), $v_6$ (mean arterial pressure (MAP)), $v_7$ (pressure weighted standard deviation ($\sigma_T$)), $v_8$ (pressure weighted mean ($\mu_2$)), $v_9$ (skewness of the arterial pulse beats ($\mu_{3P}$)), $v_{10}$ (kurtosis of the arterial pulse pressure ($\mu_{4P}$)), $v_{11}$ (pressure weighted skewness ($\mu_{3T}$)), $v_{12}$ (pressure weighted kurtosis ($\mu_{4T}$)), $v_{13}$ (pressure dependent Windkessel compliance (Cw)), and $v_{14}$ (patient body surface area (BSA)). The model was as follows:

$$\chi_i = \prod_{14} \left( \begin{bmatrix} v_1 & v_2 & \cdots & v_{14} \end{bmatrix}^{\wedge \begin{bmatrix} P_{1,1} & \cdots & P_{1,14} \\ \cdots & \cdots & \cdots \\ P_{17,1} & \cdots & P_{17,14} \end{bmatrix}} \right) \qquad \text{(Formula 14)}$$

After regression, the values of the array P (17 × 14) were determined, defining which variables are included in the model as follows:

$$P = \begin{bmatrix}
0 & 0 & 0 & 2 & 0 & 0 & 0 & 0 & 0 & 1 & 0 & 0 & 1 & 0 \\
1 & 0 & -1 & 0 & 0 & 0 & 0 & 0 & 0 & 0 & 0 & 0 & 0 & -1 \\
0 & 0 & 0 & 2 & 0 & 0 & 0 & 0 & 0 & 1 & 0 & 0 & 2 & 0 \\
0 & 0 & 0 & -2 & 0 & 0 & -1 & 0 & 0 & 1 & 0 & 0 & 0 & 0 \\
0 & 0 & 0 & 0 & 0 & 0 & 0 & 0 & 0 & -1 & 0 & 0 & 0 & 2 \\
-1 & 0 & 2 & 0 & 0 & 0 & 0 & 0 & 1 & 0 & 0 & 0 & 0 & 0 \\
1 & 0 & 0 & 0 & 0 & -2 & 0 & 0 & 0 & 0 & 0 & 0 & 2 & 0 \\
0 & 0 & 0 & -1 & 0 & 0 & -2 & 0 & 0 & 0 & 0 & 0 & 0 & 2 \\
0 & 0 & 0 & 0 & 0 & 0 & 0 & 0 & 2 & 0 & 0 & 0 & 1 & 0 \\
0 & 0 & 0 & 0 & 0 & 0 & 0 & 2 & 0 & -2 & 0 & 0 & 2 & 0 \\
0 & 0 & 0 & 1 & 0 & 0 & 0 & 1 & 0 & 0 & 0 & 0 & 2 & 0 \\
0 & 0 & 0 & 0 & 0 & 0 & 0 & 1 & 1 & -1 & 0 & 0 & 0 & 0 \\
0 & 0 & 0 & -2 & 0 & 0 & 0 & 0 & 0 & 2 & 0 & 0 & 0 & 2 \\
0 & 0 & 0 & -1 & 0 & 0 & -2 & 0 & 0 & 1 & 0 & 0 & 0 & 0 \\
0 & 0 & 0 & 0 & 0 & 1 & 0 & 0 & 2 & 0 & 0 & 0 & 0 & 2 \\
0 & 0 & 0 & 0 & 0 & 0 & 1 & 0 & 2 & -2 & 0 & 0 & 0 & 0 \\
2 & 0 & 0 & 0 & 0 & 0 & 0 & 0 & -2 & 0 & 0 & 0 & 0 & 2
\end{bmatrix}$$

The regression was performed such that the number of parameters per regression term was restrained to less than three, with each parameter having an order no greater than two. Thus, as shown above, each row of the matrix P has at most three non-zero terms, with the absolute value of each element of P being at most two. These constraints were set for the sake of numerical stability and accuracy. The expression for $\chi$ therefore became a 17-term second order curve in 14- dimensional parameter space. The resultant expression for $\chi$ can be written as:

$$\chi = \begin{bmatrix} A_1 & A_2 & ... & A_n \end{bmatrix} * \begin{bmatrix} X_1[1] \\ X_1[2] \\ ... \\ X_1[17] \end{bmatrix} \qquad \text{(Formula 15)}$$

Where $A_1$ ... $A_{17}$ are the coefficients of the polynomial multiregression model. These coefficients were then computed (using known numerical methods) to best relate the parameters to $\chi$ given the chosen suite of factors to equal +100 or -100. A polynomial multivariate fitting function based on a least-squares regression was used to generate the following coefficients for the polynomial:

$$A = \begin{bmatrix} 0.35578 \\ -1.6207 \\ -0.53381 \\ -28.818 \\ 1.996 \\ 0.41195 \\ 0.60126 \\ -107.64 \\ -1.3753 \\ -0.065631 \\ 0.18202 \\ -0.43511 \\ 0.28365 \\ 265.62 \\ -0.26331 \\ 0.81367 \\ -0.0048077 \end{bmatrix}$$

Thus, a subject's cardiovascular parameters can be determined by first creating a model as just described (i.e., determining an approximating function relating a set of clinically derived reference measurements of a model output value that corresponds to a first established value for the arterial pressure waveforms of a first set of arterial pressure waveform data and a second established value for the arterial pressure waveforms of a second set of arterial pressure waveform data, the output values representing clinical measurements of the cardiovascular parameter from both subjects not experiencing the vascular condition and subjects experiencing the vascular condition, the approximating function being a function of one or more of the parameters described above, and a set of clinically determined reference measurements representing blood pressure parameters dependent upon the cardiovascular parameter from subjects with normal hemodynamic conditions or subjects experiencing abnormal hemodynamic conditions (depending on the model)). Next determining a set of arterial blood pressure parameters from the arterial blood pressure waveform data, the set of arterial blood pressure parameters including the same parameters used to create the multivariate statistical model. Then estimating the subject's cardiovascular parameter by evaluating the approximating function with the set of arterial blood pressure parameters.

Once such a model is developed, it can be used to detect the cardiovascular condition of any subject continuously in real-time. The model can be continuously evaluated using the chosen factors determined from a subject's atrial pressure waveform. In this example, the first established value was set at +100 and the second established value was set at -100, so the threshold value could, for example, be set at zero in this model to provide:

$\chi \geq 0 \rightarrow$ vascular condition indicated

$\chi < 0 \rightarrow$ vascular condition not indicated

Also disclosed herein is a method for creating such a multivariate statistical model for use in detecting a vascular condition in a subject. This method is accomplished in a similar fashion to the example just provided. Specifically, the method includes providing a first set of arterial pressure waveform data from a first group of subjects that were experiencing the vascular condition and providing a second set of arterial pressure waveform data from a second group of subjects that were not experiencing the vascular condition. Then, the data are used to build a multivariate statistical model that provides a model output value corresponding to a first established value for the arterial pressure waveforms of the first set of arterial pressure waveform data and a second established value for the arterial pressure waveform of the second set of arterial pressure waveform data. The multivariate statistical model utilizes a set of factors including one or more parameters affected by the vascular condition as discussed above.

Further disclosed is a method for detecting a vascular condition in a subject. The method includes providing arterial pressure waveform data from a subject. This arterial pressure waveform data will be the data upon which a multivariate statistical model is based. Then applying the multivariate statistical model to the arterial pressure waveform data to determine a cardiovascular parameter. The multivariate statistical model can be prepared as described above from a first set of arterial pressure waveform data from a first group of subjects that were experiencing the vascular condition and a second set of arterial pressure waveform data from a second group of subjects that were not experiencing the vascular condition. The multivariate statistical model is set to provide a cardiovascular parameter that corresponds to a first established value for the arterial pressure waveforms of the first set of arterial pressure waveform data and a second established value for the arterial pressure waveform of the second set of arterial pressure waveform data. Once a cardiovascular parameter is calculated, the cardiovascular parameter is compared to a pre-established threshold value. The pre-established threshold value is chosen so that a cardiovascular parameter equal to or greater than the threshold value indicates the subject is experiencing the vascular condition, and a cardiovascular parameter less than the threshold value indicates the subject is not experiencing the vascular condition.

The method for detecting a vascular condition in a subject can be used to continuously monitor a subject, so that possible changes in condition over time are monitored. The method can further alert a user when the vascular condition is indicated. Such an alert can be a notice published on a graphical user interface or a sound.

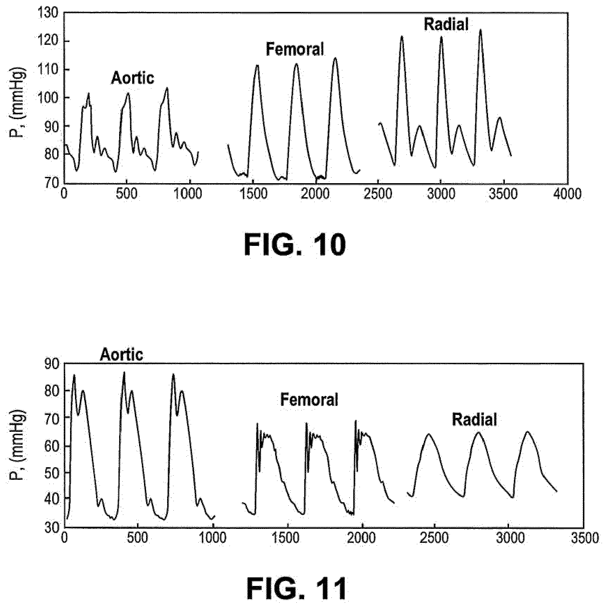

This model can be used, for example, to detect peripheral arterial pressure decoupling. As used herein, the phrase peripheral arterial pressure decoupling means a condition in which the peripheral arterial pressure and/or flow are decoupled from the central aortic pressure and/or flow, and the term peripheral arteries is intended to mean arteries located away from the heart, *e.g.,* radial, femoral, or brachial arteries. Decoupled arterial pressure means that the normal relationship between peripheral arterial pressure, and central pressure is not valid. This also includes conditions in which the peripheral arterial pressure is not proportional or is not a function of the central aortic pressure. Under normal hemodynamic conditions, blood pressure increases the further away from the heart the measurement is taken. Such a pressure increase is shown in Fig. 10, *i.e.,* the amplitude of a pressure wave measured at radial arteries is greater than the pressure measured at the femoral artery, which in turn is greater than the aortic pressure. These differences in pressure are related to wave reflection, *i.e.,* pressure is amplified toward the periphery.

This normal hemodynamic relationship of pressures, *i.e.,* an increase in pressure away from the heart, is often relied upon in medical diagnosis. However, under hyperdynamic conditions, this relationship can become inverted with the arterial pressure becoming lower than the central aortic pressure. This reversal has been attributed, for example, to arterial tone in the peripheral vessels, which is suggested to impact the wave reflections discussed above. Such a hyperdynamic condition is shown in Fig. 11, *i.e.,* the amplitude of a pressure wave measured at radial arteries is lower than the pressure measured as the femoral artery, which in turn is lower than the aortic pressure. Drugs that dilate small peripheral arteries (e.g., nitrates, ACE inhibitors, and calcium inhibitors) are thought to contribute to hyperdynamic conditions. These types of severe vasodilatory conditions are also often observed in situations right after cardiopulmonary bypass (coronary bypass), in which the radial arterial pressure underestimates the pressure in the aorta. Substantial central to peripheral pressure differences, where the peripheral arterial pressure underestimates the central aortic pressure, are usually observed in patients with severe sepsis who are treated with large amount of fluids and high-dose vasopressors used to treat the severe vasodilation. Very similar conditions are also observed in patient with end stage liver disease. As will be well appreciated by those of skill in the art, certain treatments for subjects in normal hemodynamic conditions will be approached differently than for subjects in hyperdynamic conditions. Thus, the presently disclosed methods for detecting vascular conditions such as vasodilation in a subject will be very useful to those of skill in the art.

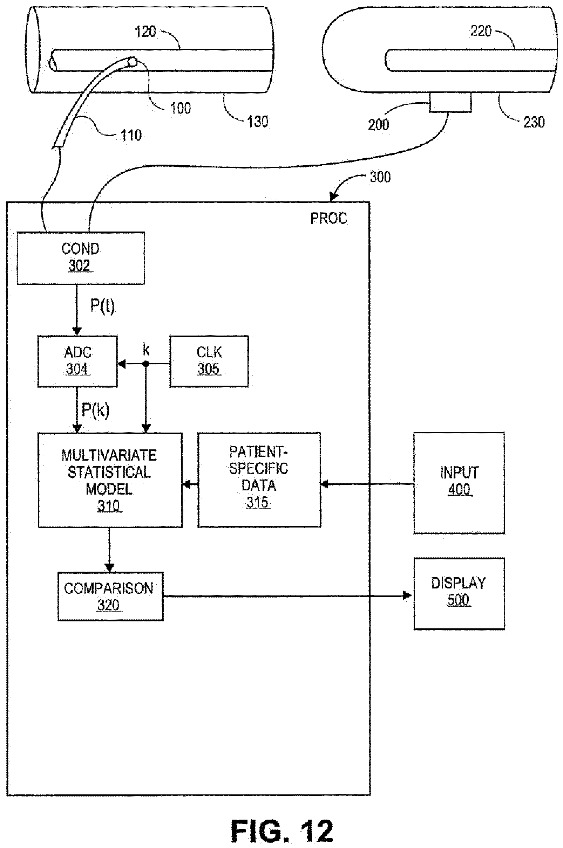

Fig. 12 shows the main components of a system that implements the methods described herein for detecting vascular conditions such as vasodilation in a subject. The methods may be implemented within an existing patient-monitoring device, or it may be implemented as a dedicated monitor. As is mentioned above, pressure, or some other input signal proportional to, derived from, or a function of pressure, may be sensed in either or, indeed, both, of two ways: invasively and non-invasively. For convenience, the system is described as measuring arterial blood pressure as opposed to some other input signal that is converted to pressure.

Fig. 12 shows both types of pressure sensing for the sake of completeness. In most practical applications of the methods described herein, either one or several variations will typically be implemented. In invasive applications of the methods described herein, a conventional pressure sensor 100 is mounted on a catheter 110, which is inserted in an artery 120 of a portion 130 of the body of a human or animal patient. The artery 120 is any artery in the arterial system, such as, for example, the femoral, radial or brachial artery. In the non-invasive applications of the methods described herein, a conventional pressure sensor 200, such as a photo-plethysmographic blood pressure probe, is mounted externally in any conventional manner, for example using a cuff around a finger 230 or a transducer mounted on the wrist of the patient. Fig. 12 schematically shows both types.

The signals from the sensors 100, 200 are passed via any known connectors as inputs to a processing system 300, which includes one or more processors and other supporting hardware and system software (not shown) usually included to process signals and execute code. The methods described herein may be implemented using a modified, standard, personal computer, or may be incorporated into a larger, specialized monitoring system. For use with the methods described herein, the processing system 300 also may include, or is connected to, conditioning circuitry 302 which performs normal signal processing tasks such as amplification, filtering, or ranging, as needed. The conditioned, sensed input pressure signal P(t) is then converted to digital form by a conventional analog-to-digital converter ADC 304, which has or takes its time reference from a clock circuit 305. As is well understood, the sampling frequency of the ADC 304 should be chosen with regard to the Nyquist criterion so as to avoid aliasing of the pressure signal (this procedure is very well known in the art of digital signal processing). The output from the ADC 304 will be the discrete pressure signal P(k), whose values may be stored in conventional memory circuitry (not shown).

The values P(k) are passed to or accessed from memory by a software module 310 comprising computer-executable code for implementing whichever multivariate statistical model is chosen for calculating a vascular condition. The design of such a software module 310 will be straight forward to one of skill in computer programming.

If used, patient-specific data such as age, height, weight, BSA, etc., is stored in a memory region 315, which may also store other predetermined parameters such as threshold or threshold range values. These values may be entered using any known input device 400 in the conventional manner.

Comparison of a calculated cardiovascular parameter to a threshold value is done in module 320. Calculation module 320 includes computer-executable code and take as inputs the output of module 310 and threshold value information, then performs the chosen calculations for determining if a subject is experiencing a vascular condition.

As illustrated by Fig. 12, the comparison results may be passed to any conventional display or recording device 500 for presentation to and interpretation by a user. As with the input device 400, the display 500 will typically be the same as is used by the processing system for other purposes.

For each of the methods described herein, when the vascular condition is detected, a user can be notified of the vascular condition. The user can be notified of the vasodilatory conditions by publishing a notice on display 500 or another graphical user interface device. Further, a sound can be used to notify the user of the vascular condition. Both visual and auditory signals can be used.

Exemplary embodiments of the present invention have been described above with reference to a block diagram and a flowchart illustration of methods, apparatuses, and computer program products. One of skill will understand that each block of the block diagram and flowchart illustration, and combinations of blocks in the block diagram and flowchart illustration, respectively, can be implemented by various means including computer program instructions. These computer program instructions may be loaded onto a general purpose computer, special purpose computer, or other programmable data processing apparatus to produce a machine, such that the instructions which execute on the computer or other programmable data processing apparatus create a means for implementing the functions specified in the flowchart block or blocks.

The methods described herein further relate to computer program instructions that may be stored in a computer-readable memory that can direct a computer or other programmable data processing apparatus, such as in a processor or processing system (shown as 300 in Fig. 12), to function in a particular manner, such that the instructions stored in the computer-readable memory produce an article of manufacture including computer-readable instructions for implementing the function specified in the blocks illustrated in Fig. 12. The computer program instructions may also be loaded onto a computer, the processing system 300, or other programmable data processing apparatus to cause a series of operational steps to be performed on the computer, the processing system 300, or other programmable apparatus to produce a computer-implemented process such that the instructions that execute on the computer or other programmable apparatus provide steps for implementing the functions specified in the blocks. Moreover, the various software modules 310 and 320 used to perform the various calculations and perform related method steps described herein also can be stored as computer-executable instructions on a computer-readable medium in order to allow the methods to be loaded into and executed by different processing systems.

Accordingly, blocks of the block diagram and flowchart illustration support combinations of means for performing the specified functions, combinations of steps for performing the specified functions, and program instruction means for performing the specified functions. One of skill will understand that each block of the block diagram and flowchart illustration, and combinations of blocks in the block diagram and flowchart illustration, can be implemented by special purpose hardware-based computer systems that perform the specified functions or steps, or combinations of special purpose hardware and computer instructions.

The present invention is not limited in scope by the embodiments disclosed herein which are intended as illustrations of a few aspects of the invention and any embodiments which are functionally equivalent are within the scope of this invention as defined by the appended claims. Various modifications of the models and methods in addition to those shown and described herein will become apparent to those skilled in the art and are intended to fall within the scope of the appended claims. Further, while only certain representative combinations of the models and method steps disclosed herein are specifically discussed in the embodiments above, other combinations of the model components and method steps will become apparent to those skilled in the art and also are intended to fall within the scope of the appended claims. Thus a combination of components or steps may be explicitly mentioned herein; however, other combinations of components and steps are included, even though not explicitly stated. The term "comprising" and variations thereof as used herein is used synonymously with the term "including" and variations thereof and are open, non-limiting terms.

In the following, further embodiments not being part of the invention are disclosed: In a further embodiment, a model for use in detecting a vascular condition in a subject comprises:

a multivariate statistical model based on arterial pressure waveform data from a first group of subjects that were experiencing the vascular condition and a second group of subjects that were not experiencing the vascular condition, the multivariate statistical model providing a model output value that corresponds to a first established value for the arterial pressure waveforms of the first set of arterial pressure waveform data and a second established value for the arterial pressure waveforms of the second set of arterial pressure waveform data.

Additionally, in another embodiment of the model, the multivariate statistical model is based on a set of factors including one or more parameters affected by the vascular condition.

In a further embodiment of the model, the one or more parameters affected by the vascular condition are selected from the group consisting of (a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data.

Furthermore, in another embodiment of the method, the one or more parameters affected by the vascular condition include (a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data.

Moreover, a further embodiment the method comprises detecting the vascular condition additionally using one or more of (n) a parameter based on the shape of the beat-to-beat arterial blood pressure signal and at least one statistical moment of the arterial blood pressure signal having an order of one or greater, (o) a parameter corresponding to the heart rate, and (p) a set of anthropometric parameters of the subject.

Additionally, in another embodiment of the method, the first established value is greater than the second established value.

Apart from that, in a further embodiment of the method, the first established value is a positive number and the second established value is a negative number.

Aside from that, in another embodiment of the method, the first established value is +100 and the second established value is -100.

Furthermore, a further embodiment of a method for creating a model for use in detecting a vascular condition in a subject comprises:

providing a first set of arterial pressure waveform data from a first group of subjects that were experiencing the vascular condition;

providing a second set of arterial pressure waveform data from a second group of subjects that were not experiencing the vascular condition; and

building a multivariate statistical model based on the first and second sets of arterial pressure waveform data, the multivariate statistical model providing a model output value that corresponds to a first established value for the arterial pressure waveforms of the first set of arterial pressure waveform data and a second established value for the arterial pressure waveform of the second set of arterial pressure waveform data.

Moreover, in another embodiment of the method, the multivariate statistical model is based on a set of factors including one or more parameters affected by the vascular condition.

In yet a further embodiment of the method, the one or more parameters affected by the vascular condition are selected from the group consisting of (a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data.

In addition to that, in another embodiment of the method, the one or more parameters affected by the vascular condition include (a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data.

Apart from that, a further embodiment of the method comprises detecting the vascular condition additionally using one or more of (d) a parameter based on the shape of the beat-to-beat arterial blood pressure signal and at least one statistical moment of the arterial blood pressure signal having an order of one or greater, (e) a parameter corresponding to the heart rate, and (f) a set of anthropometric parameters of the subject.

Additionally, in another embodiment of the method, the first established value is greater than the second established value.

Moreover, in a further embodiment of the method, the first established value is a positive number and the second established value is a negative number.

Furthermore, in another embodiment of the method, the fist established value is +100 and the second established value is -100.

Aside from that, in a further embodiment of the method, building a multivariate statistical model comprises:

determining an approximating function relating a set of clinically determined reference measurements of a model output value that corresponds to a first established value for the arterial pressure waveforms of the first set of arterial pressure waveform data and a second established value for the arterial pressure waveform of the second set of arterial pressure waveform data, the output values representing clinical measurements of the cardiovascular parameter from both subjects not experiencing the vascular condition and subjects experiencing the vascular condition, the approximating function being a function of one or more of the following parameters(a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data;

determining a set of arterial blood pressure parameters from the arterial blood pressure waveform data, the set of arterial blood pressure parameters including one or more of the following parameters(a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data; and

estimating the model output value by evaluating the approximating function with the set of the parameters.

A further embodiment of a method of detecting a vascular condition in a subject comprises:

providing arterial pressure waveform data from the subject;
applying a multivariate statistical model to the arterial pressure waveform data to determine a cardiovascular parameter, the multivariate statistical model being prepared from a first set of arterial pressure waveform data from a first group of subjects that were experiencing the vascular condition and a second set of arterial pressure waveform data from a second group of subjects that were not experiencing the vascular condition, the multivariate statistical model providing a cardiovascular parameter that corresponds to a first established value for the arterial pressure waveforms of the first set of arterial pressure waveform data and a second established value for the arterial pressure waveform of the second set of arterial pressure waveform data; and
comparing the cardiovascular parameter to a threshold value,
wherein a cardiovascular parameter equal to or greater than the threshold value indicates the subject is experiencing the vascular condition, and a cardiovascular parameter less than the threshold value indicates the subject is not experiencing the vascular condition.

Moreover, in another embodiment of the method, the multivariate statistical model is based on a set of factors including one or more parameters affected by the vascular condition.

Apart from that, in a further embodiment of the method, the one or more parameters affected by the vascular condition are selected from the group consisting of (a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data.

In addition to that, in another embodiment of the method, the one or more parameters affected by the vascular condition include (a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data.

In an another embodiment of the method, the first established value is greater than the second established value.

Moreover, in a further embodiment of the method, the first established value is a positive number and the second established value is a negative number.

Furthermore, in another embodiment of the method, the first established value is +100 and the second established value is -100.

Aside from that, in a further embodiment of the method, the threshold value is 0.

Additionally, in another embodiment of the method, the threshold value is the mean of the first established value and the second established value.

Furthermore, in a further embodiment of the method, the threshold value is a threshold range, and if the cardiovascular parameter is within the threshold range the result is indeterminate and the method is repeated using additional arterial pressure waveform data from the subject.

In addition to that, in another embodiment of the method, the multivariate statistical model is created using the following steps:

determining an approximating function relating a set of clinically determined reference measurements of a model output value that corresponds to a first established value for the arterial pressure waveforms of the first set of arterial pressure waveform data and a second established value for the arterial pressure waveform of the second set of arterial pressure waveform data, the output values representing clinical measurements of the cardiovascular parameter from both subjects not experiencing the vascular condition and subjects experiencing the vascular condition, the approximating function being a function of one or more of the following parameters(a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data;
determining a set of arterial blood pressure parameters from the arterial blood pressure waveform data, the set of arterial blood pressure parameters including one or more of the following parameters(a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (1) a parameter based on the pressure weighted kurtosis of a set of arterial pressure waveform data, and (m) a parameter based on the pressure dependent Windkessel compliance of a set of arterial pressure waveform data; and
estimating the model output value by evaluating the approximating function with the set of the parameters.

Furthermore, in a further embodiment of the method, the arterial pressure waveform data from the subject is continuously provided and continuously analyzed.

Additionally, a further embodiment of the method comprises alerting a user when the vascular condition is detected.

In a further embodiment of the method, the user is alerted by publishing a notice on a graphical user interface.

Finally, in another embodiment of the method, a user is alerted by emitting a sound.

## Claims

1.  System for creating a model for use in detecting a peripheral arterial pressure decoupling in a subject, the system comprising:

    means for providing a first set of arterial pressure waveform data from a first group of subjects that were experiencing the peripheral arterial pressure decoupling;
    means for providing a second set of arterial pressure waveform data from a second group of subjects that were not experiencing the peripheral arterial pressure decoupling; and
    means for building a multivariate Boolean model based on the first and second sets of arterial pressure waveform data, the multivariate Boolean model utilizing a model output value that corresponds to a first established value for the arterial pressure waveforms of the first set of arterial pressure waveform data and a second established value for the arterial pressure waveform of the second set of arterial pressure waveform data, **characterized in that** the multivariate Boolean model is built so as to minimize a first mean square error between an output value calculated for each subject of the first group of subjects and the first established value, and to minimize a second mean square error between an output value calculated for each subject of the second group of subjects and the second established value.

2.  System for detecting peripheral arterial pressure decoupling in a subject, the system comprising:

    means (100, 200) for providing arterial pressure waveform data from the subject; means (300) for applying a multivariate Boolean model to the arterial pressure waveform data to determine a cardiovascular parameter, the multivariate Boolean model being prepared from a first set of arterial pressure waveform data from a first group of subjects that were experiencing the peripheral arterial pressure decoupling, and a second set of arterial pressure waveform data from a second group of subjects that were not experiencing the peripheral arterial pressure decoupling, the multivariate Boolean model providing a cardiovascular parameter that corresponds to a first established value for the arterial pressure waveforms of the first set of arterial pressure waveform data and a second established value for the arterial pressure waveform of the second set of arterial pressure waveform data, wherein the multivariate Boolean model has been built so as to minimize a first mean square error between an output value calculated for each subject of the first group of subjects and the first established value, and to minimize a second mean square error between an output value calculated for each subject of the second group of subjects and the second established value; and
    means (320) for comparing the cardiovascular parameter to a threshold value, wherein a cardiovascular parameter equal to or greater than the threshold value indicates the subject is experiencing the peripheral arterial pressure decoupling, and a cardiovascular parameter less than the threshold value indicates the subject is not experiencing the peripheral arterial pressure decoupling.

## Patentansprüche

1.  System zum Erstellen eines Modells zur Verwendung beim Detektieren einer peripheren Arteriendruckentkopplung bei einem Probanden, wobei das System umfasst:

    Mittel zum Bereitstellen eines ersten Satzes von Arteriendruck-Wellenformdaten aus einer ersten Gruppe von Probanden, bei denen die periphere Arteriendruckentkopplung auftrat;
    Mittel zum Bereitstellen eines zweiten Satzes von Arteriendruck-Wellenformdaten aus einer zweiten Gruppe von Probanden, bei denen die periphere Arteriendruckentkopplung nicht auftrat; und
    Mittel zum Erstellen eines multivariaten Booleschen Modells basierend auf dem ersten und dem zweiten Satz von Arteriendruck-Wellenformdaten, wobei das mulitvariate Boolesche Modell einen Modell-Ausgabewert verwendet, der einem ersten festgelegten Wert für die Arteriendruck-Wellenformen des ersten Satzes von Arteriendruck-Wellenformdaten und einem zweiten festgelegten Wert für die Arteriendruck-Wellenform des zweiten Satzes von Arteriendruck-Wellenformdaten entspricht, **dadurch gekennzeichnet, dass** das multivariate Boolesche Modell derart aufgebaut ist, dass es einen ersten mittleren quadratischen Fehler zwischen einem für jeden Probanden aus der ersten Gruppe von Probanden berechneten Ausgabewert und dem ersten festgelegten Wert minimiert, und dass es einen zweiten mittleren quadratischen Fehler zwischen einem für jeden Probanden aus der zweiten Gruppe von Probanden berechneten Ausgabewert und dem zweiten festgelegten Wert minimiert.

**2.** System zum Detektieren einer peripheren Arteriendruckentkopplung bei einem Probanden, wobei das System umfasst:

Mittel (100, 200) zum Bereitstellen von Arteriendruck-Wellenformdaten von dem Probanden;
Mittel (300) zum Anwenden eines multivariaten Booleschen Modells auf die Arteriendruck-Wellenformdaten zur Bestimmung eines kardiovaskulären Parameters, wobei das multivariate Boolesche Modell aus einem ersten Satz von Arteriendruck-Wellenformdaten von einer ersten Gruppe von Probanden, bei denen die periphere Arteriendruckentkopplung auftrat, und einem zweiten Satz von Arteriendruck-Wellenformdaten von einer zweiten Gruppe von Probanden, bei denen die periphere Arteriendruckentkopplung nicht auftrat, erstellt wird, wobei das multivariate Boolesche Modell einen kardiovaskulären Parameter bereitstellt, der einem ersten festgelegten Wert für die Arteriendruck-Wellenformen des ersten Satzes von Arteriendruck-Wellenformdaten und einem zweiten festgelegten Wert für die Arteriendruck-Wellenform des zweiten Satzes von Arteriendruck-Wellenformdaten entspricht, wobei das multivariate Boolesche Modell derart aufgebaut ist, dass es einen ersten mittleren quadratischen Fehler zwischen einem für jeden Probanden aus der ersten Gruppe von Probanden berechneten Ausgabewert und dem ersten festgelegten Wert minimiert, und dass es einen zweiten mittleren quadratischen Fehler zwischen einem für jeden Probanden aus der zweiten Gruppe von Probanden berechneten Ausgabewert und dem zweiten festgelegten Wert minimiert; und
Mittel (320) zum Vergleichen des kardiovaskulären Parameters mit einem Schwellenwert, wobei durch einen kardiovaskulären Parameter, der gleich dem Schwellenwert ist oder diesen überschreitet, angezeigt wird, dass bei dem Probanden die periphere Arteriendruckentkopplung auftritt, und durch einen kardiovaskulären Parameter, der kleiner als der Schwellenwert ist, angezeigt wird, dass bei dem Probanden die periphere Arteriendruckentkopplung nicht auftritt.

**Revendications**

**1.** Système permettant de créer un modèle destiné à être utilisé dans la détection d'un découplage de la tension artérielle périphérique chez un sujet, ledit système comprenant :

des moyens permettant de fournir un premier jeu de données définissant la forme de l'onde de pression artérielle obtenu dans un premier groupe de sujets présentant le découplage de la tension artérielle périphérique ;
des moyens permettant de fournir un deuxième jeu de données définissant la forme de l'onde de pression artérielle obtenu dans un deuxième groupe de sujets ne présentant pas ledit découplage de la tension artérielle périphérique ; et
des moyens permettant de créer un modèle multivarié de type booléen basé sur le premier et le deuxième jeu de données définissant la forme de l'onde de pression artérielle, le modèle multivarié de type booléen utilisant une valeur de sortie modélisée laquelle correspond à une première valeur déterminée pour les formes d'onde de pression artérielle du premier jeu de données définissant la forme de l'onde de pression artérielle et à une deuxième valeur déterminée pour la forme d'onde de pression artérielle du deuxième jeu de données définissant la forme de l'onde de pression artérielle,
**caractérisé en ce que** le modèle multivarié de type booléen est conçu pour minimiser une première erreur quadratique moyenne entre une valeur de sortie calculée pour chaque sujet du premier groupe de sujets et la première valeur déterminée et pour minimiser une deuxième erreur quadratique moyenne entre une valeur de sortie calculée pour chaque sujet du deuxième groupe de sujets et la deuxième valeur déterminée.

**2.** Système permettant de détecter un découplage de la tension artérielle périphérique chez un sujet, ledit système comprenant :

des moyens (100, 200) permettant de fournir des données définissant la forme de l'onde de la pression artérielle du sujet ;
des moyens (300) permettant d'appliquer un modèle multivarié de type booléen sur les données définissant la forme de l'onde de pression artérielle en vue de déterminer un paramètre cardiovasculaire, le modèle multivarié de type booléen étant créé à partir d'un premier jeu de données définissant la forme de l'onde de pression artérielle obtenu dans un premier groupe de sujets présentant le découplage de la pression artérielle périphérique et à partir d'un deuxième jeu de données définissant la forme de l'onde de pression artérielle obtenu dans un deuxième groupe de sujets ne présentant pas ledit découplage de la pression artérielle périphérique, le modèle multivarié de type booléen fournissant un paramètre cardiovasculaire lequel correspond à une première valeur déterminée pour les formes d'onde de pression artérielle du premier jeu de données définissant la forme de l'onde de pression artérielle ainsi qu'à une deuxième valeur déterminée pour la forme d'onde de pression artérielle du deuxième jeu de données définissant la forme de l'onde de pression artérielle, le modèle multivarié de type booléen étant conçu pour minimiser une première erreur quadratique moyenne entre une valeur de sortie calculée pour chaque sujet du premier groupe de sujets et la première valeur déterminée et pour minimiser une deuxième erreur quadratique moyenne entre une valeur de sortie calculée pour chaque sujet du deuxième groupe de sujets et la deuxième valeur déterminée ; et

des moyens (320) permettant de comparer le paramètre cardiovasculaire à une valeur seuil, un paramètre cardiovasculaire qui est égal à la valeur seuil ou dépasse cette dernière indiquant que le sujet présente le découplage de la pression artérielle périphérique, et un paramètre cardiovasculaire qui est inférieur à la valeur de seuil indiquant que le sujet ne présente pas ledit découplage de la pression artérielle périphérique.

**FIG. 1**

**FIG. 2**

**FIG. 3**

**FIG. 4**

**FIG. 5**

**FIG. 6**

**FIG. 7**

**FIG. 8**

$t_{sys}$

# FIG. 9

**FIG. 10**

**FIG. 11**

**FIG. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 89088704

- US 890887

**Non-patent literature cited in the description**

- **BENJAMIN PRATT et al.** Calculating Arterial Pressure-Based Cardiac Output Using a Novel Measurement and Analysis Method. *Biomedical Instrumentation & Technology,* 12 October 2007, vol. 41 (5), 403-411

- **LANGEWOUTERS et al.** The Static Elastic Properties of 45 Human Thoracic and 20 Abnormal Aortas in vitro and the Parameters of a New Model. *J. Biomechanics,* 1984, vol. 17 (6), 425-435